# EUROPEAN PATENT APPLICATION

(11) **EP 4 644 546 A1**
(43) Date of publication of application: **05.11.2025**
(21) Application number: 24759518.4
(22) Date of filing: 02.02.2024
(51) Int. Cl.: C12N 15/113, C12N 15/84, C12Q 1/6895

(54) **METHOD FOR CREATING SOYBEANS WITH REDUCED BEANY FLAVOR**

(30) Priority: 20.02.2023 CN 202310146847
(71) Applicant: Shandong Shunfeng Biotechnology Co., Ltd., Jinan, Shandong 250000 (CN)
(72) Inventor: XIE, Hongtao, Jinan, Shandong 250000 (CN)
(74) Representative: Bayramoglu et al.
(86) International application number: PCT/CN2024/075467
(87) International publication number: WO 2024/174841

(57) **Abstract**

The present disclosure discloses a method for creating a soybean with a reduced beany flavor. The method can lower a content of a beany-flavor compound in a soybean. The simultaneous inhibition on the expression of lipoxygenase and fatty acid desaturase genes or proteins encoded thereby can significantly reduce the content of a beany-flavor compound in a plant or reduce the beany flavor of the plant, especially the beany flavor of soybean.

## Description

The present application claims priority to Chinese Patent Application CN202310146847.X filed on February 20, 2023, which is incorporated herein by reference in its entirety.

### TECHNICAL FIELD

The present disclosure relates to the field of biotechnologies, and specifically relates to a method for creating a soybean with a reduced beany flavor. The soybean developed by the present disclosure enables a reduced beany odor.

### BACKGROUND TECHNOLOGY

Soybean is an important source for fats and proteins in the human, and is also one of the most significant cash crops worldwide. Soybeans and soy protein are widely used to produce foods such as soybean milk, plant-based milk, tofu, tofu pudding, dried tofu, yuba, sausages, and cooked meats due to various advantages such as comprehensive nutrition, high protein content, and low cholesterol content. However, the beany-flavor compounds in soybeans, such as hexanal, 2-hexenal, 1-hexanol, 1-octen-3-ol, *trans*-2-octenal, nonanal, and *trans*-2-nonenal, severely affect the flavors of soy products and soy protein products and heavily limit the application of soybeans and soy protein powders in the food industry. For example, due to the beany flavor, a soy protein powder content is restricted to no more than 8% in meat products and to no more than 3% in baked goods. The beany flavor also greatly hinders the application of soybeans and soy protein powders in ice creams, fish meat-based products, whipped cream for decorating, plant protein drinks, infant milk powders, meal replacement powders, soy yogurt, foods for special medical purposes, etc.

In recent decades, to address the urgent demand for soybeans and soy protein powders with reduced or no beany flavor in the food industry, lipoxygenase-free soybean varieties with low beany flavor have been developed as soybean raw materials. Studies have shown that the complete loss of functions of lipoxygenases Lox1/2/3 can reduce the contents of beany-flavor compounds such as hexanal, 1-hexanol, *trans*-2-octenal, nonanal, and *trans*-2-nonenal by about 40% to 70% and the content of 1-octen-3-ol by about 20% in soybeans, but does not make the content of 2-hexenal significantly change. Researchers have successfully utilized soybeans with lipoxygenases LOX1/2/3 deficient in the production of ice creams, blended plant-based milk, and other products. However, these low-beany-flavor soybeans still include significant amounts of beany-flavor compounds including hexanal, 2-hexenal, 1-hexanol, 1-octen-3-ol, *trans*-2-octenal, nonanal, and *trans*-2-nonenal, which severely affects the application range of low-beany-flavor soybeans currently on the market. Therefore, there is an urgent market demand for soybeans without a beany flavor, which means that the contents of beany-flavor compounds such as hexanal, 1-hexanol, *trans-2-octenal,* nonanal, and *trans*-2-nonenal should be reduced by 90% or more and the contents of 2-hexenal and 1-octen-3-ol should be reduced to even lower levels.

The soybean seeds acquired through the CRISPR/Cas gene editing technology in the present disclosure are nearly free of beany-flavor compounds including hexanal, 1-hexanol, *trans-2-octenal,* nonanal, and *trans*-2-nonenal, and have the contents of 2-hexenal and l-octen-3-ol remarkably reduced. Thus, the soybean seeds acquired in the present disclosure have a promising application prospect.

### CONTENT OF THE INVENTION

An objective of the present disclosure is to provide a method for reducing a content of a beany-flavor compound in a plant or reducing a beany flavor of the plant.

A first aspect of the present disclosure provides a method for reducing a content of a beany-flavor compound in a plant or reducing a beany flavor of the plant, including a step of reducing or inhibiting expression levels and/or activities of lipoxygenase and fatty acid desaturase genes or proteins encoded thereby in the plant.

In another preferred embodiment, the lipoxygenase gene is selected from any one or more of a Lox1 gene, a Lox2 gene, or a Lox3 gene.

In a preferred embodiment, the method includes reducing or inhibiting expression levels and/or activities of a Lox1 gene, a Lox2 gene, and a Lox3 gene or proteins encoded thereby.

In another preferred embodiment, the fatty acid desaturase gene is selected from an FAD2-1A gene and/or an FAD2-1B gene.

In a preferred embodiment, the method includes reducing or inhibiting expression levels and/or activities of an FAD2-1A gene and an FAD2-1B gene or proteins encoded thereby.

In another preferred embodiment, an amino acid sequence of a protein encoded by the FAD2-1A gene is selected from the group consisting of the following:
(i) a polypeptide with an amino acid sequence shown in SEQ ID NO.: 1;
(ii) a polypeptide that is produced through substitution, deletion, or addition of one or more (such as 1 to 10) amino acid residues in the amino acid sequence shown in SEQ ID NO.: 1 and has the same or similar function as or to the polypeptide defined in (i); or
(iii) a polypeptide that has an amino acid sequence possessing a homology of 50% or more (preferably 60% or more, 70% or more, and 80% or more, more preferably 90% or more, even more preferably 95% or more, and most preferably 98% or more, such as 99% or 100%) with the amino acid sequence shown in SEQ ID NO.: 1 and exhibits the same or similar function as or to the amino acid sequence shown in SEQ ID NO.: 1.

In another preferred embodiment, an amino acid sequence of a protein encoded by the FAD2-1B gene is selected from the group consisting of the following:
(i) a polypeptide with an amino acid sequence shown in SEQ ID NO.: 2;
(ii) a polypeptide that is produced through substitution, deletion, or addition of one or more (such as 1 to 10) amino acid residues in the amino acid sequence shown in SEQ ID NO.: 2 and has the same or similar function as or to the polypeptide defined in (i); or
(iii) a polypeptide that has an amino acid sequence possessing a homology of 50% or more (preferably 60% or more, 70% or more, and 80% or more, more preferably 90% or more, even more preferably 95% or more, and most preferably 98% or more, such as 99% or 100%) with the amino acid sequence shown in SEQ ID NO.: 2 and exhibits the same or similar function as or to the amino acid sequence shown in SEQ ID NO.: 2.

In another preferred embodiment, an amino acid sequence of a protein encoded by the Lox1 gene is selected from the group consisting of the following:
(i) a polypeptide with an amino acid sequence shown in SEQ ID NO.: 3;
(ii) a polypeptide that is produced through substitution, deletion, or addition of one or more (such as 1 to 10) amino acid residues in the amino acid sequence shown in SEQ ID NO.: 3 and has the same or similar function as or to the polypeptide defined in (i); or
(iii) a polypeptide that has an amino acid sequence possessing a homology of 50% or more (preferably 60% or more, 70% or more, and 80% or more, more preferably 90% or more, even more preferably 95% or more, and most preferably 98% or more, such as 99% or 100%) with the amino acid sequence shown in SEQ ID NO.: 3 and exhibits the same or similar function as or to the amino acid sequence shown in SEQ ID NO.: 3.

In another preferred embodiment, an amino acid sequence of a protein encoded by the Lox2 gene is selected from the group consisting of the following:
(i) a polypeptide with an amino acid sequence shown in SEQ ID NO.: 4;
(ii) a polypeptide that is produced through substitution, deletion, or addition of one or more (such as 1 to 10) amino acid residues in the amino acid sequence shown in SEQ ID NO.: 4 and has the same or similar function as or to the polypeptide defined in (i); or
(iii) a polypeptide that has an amino acid sequence possessing a homology of 50% or more (preferably 60% or more, 70% or more, and 80% or more, more preferably 90% or more, even more preferably 95% or more, and most preferably 98% or more, such as 99% or 100%) with the amino acid sequence shown in SEQ ID NO.: 4 and exhibits the same or similar function as or to the amino acid sequence shown in SEQ ID NO.: 4.

In another preferred embodiment, an amino acid sequence of a protein encoded by the Lox3 gene is selected from the group consisting of the following:
(i) a polypeptide with an amino acid sequence shown in SEQ ID NO.: 5;
(ii) a polypeptide that is produced through substitution, deletion, or addition of one or more (such as 1 to 10) amino acid residues in the amino acid sequence shown in SEQ ID NO.: 5 and has the same or similar function as or to the polypeptide defined in (i); or
(iii) a polypeptide that has an amino acid sequence possessing a homology of 50% or more (preferably 60% or more, 70% or more, and 80% or more, more preferably 90% or more, even more preferably 95% or more, and most preferably 98% or more, such as 99% or 100%) with the amino acid sequence shown in SEQ ID NO.: 5 and exhibits the same or similar function as or to the amino acid sequence shown in SEQ ID NO.: 5.

In another preferred embodiment, a nucleotide sequence of the FAD2-1A gene is selected from the group consisting of the following:
(a) a polynucleotide encoding the polypeptide shown in SEQ ID NO.: 1;
(b) a polynucleotide with a sequence shown in SEQ ID NO.: 6;
(c) a polynucleotide that has a nucleotide sequence possessing a homology of 95% or more (preferably 98% or more and more preferably 99% or more) with the sequence shown in SEQ ID NO.: 6;
(d) a polynucleotide produced by truncating or adding 1 to 60 (preferably 1 to 30 and more preferably 1 to 10) nucleotides to a 5' terminus and/or a 3' terminus of the polynucleotide shown in SEQ ID NO.: 6; and
(e) a polynucleotide complementary to a polynucleotide shown in any one of (a) to (d).

In another preferred embodiment, a nucleotide sequence of the FAD2-1B gene is selected from the group consisting of the following:
(a) a polynucleotide encoding the polypeptide shown in SEQ ID NO.: 2;
(b) a polynucleotide with a sequence shown in SEQ ID NO.: 7;
(c) a polynucleotide that has a nucleotide sequence possessing a homology of 95% or more (preferably 98% or more and more preferably 99% or more) with the sequence shown in SEQ ID NO.: 7;
(d) a polynucleotide produced by truncating or adding 1 to 60 (preferably 1 to 30 and more preferably 1 to 10) nucleotides to a 5' terminus and/or a 3' terminus of the polynucleotide shown in SEQ ID NO.: 7; and
(e) a polynucleotide complementary to a polynucleotide shown in any one of (a) to (d).

In another preferred embodiment, a nucleotide sequence of the Lox1 gene is selected from the group consisting of the following:
(a) a polynucleotide encoding the polypeptide shown in SEQ ID NO.: 3;
(b) a polynucleotide with a sequence shown in SEQ ID NO.: 8;
(c) a polynucleotide that has a nucleotide sequence possessing a homology of 95% or more (preferably 98% or more and more preferably 99% or more) with the sequence shown in SEQ ID NO.: 8;
(d) a polynucleotide produced by truncating or adding 1 to 60 (preferably 1 to 30 and more preferably 1 to 10) nucleotides to a 5' terminus and/or a 3' terminus of the polynucleotide shown in SEQ ID NO.: 8; and
(e) a polynucleotide complementary to a polynucleotide shown in any one of (a) to (d).

In another preferred embodiment, a nucleotide sequence of the Lox2 gene is selected from the group consisting of the following:
(a) a polynucleotide encoding the polypeptide shown in SEQ ID NO.: 4;
(b) a polynucleotide with a sequence shown in SEQ ID NO.: 9;
(c) a polynucleotide that has a nucleotide sequence possessing a homology of 95% or more (preferably 98% or more and more preferably 99% or more) with the sequence shown in SEQ ID NO.: 9;
(d) a polynucleotide produced by truncating or adding 1 to 60 (preferably 1 to 30 and more preferably 1 to 10) nucleotides to a 5' terminus and/or a 3' terminus of the polynucleotide shown in SEQ ID NO.: 9; and
(e) a polynucleotide complementary to a polynucleotide shown in any one of (a) to (d).

In another preferred embodiment, a nucleotide sequence of the Lox3 gene is selected from the group consisting of the following:
(a) a polynucleotide encoding the polypeptide shown in SEQ ID NO.: 5;
(b) a polynucleotide with a sequence shown in SEQ ID NO.: 10;
(c) a polynucleotide that has a nucleotide sequence possessing a homology of 95% or more (preferably 98% or more and more preferably 99% or more) with the sequence shown in SEQ ID NO.: 10;
(d) a polynucleotide produced by truncating or adding 1 to 60 (preferably 1 to 30 and more preferably 1 to 10) nucleotides to a 5' terminus and/or a 3' terminus of the polynucleotide shown in SEQ ID NO.: 10; and
(e) a polynucleotide complementary to a polynucleotide shown in any one of (a) to (d).

In another preferred embodiment, the amino acid sequence of the protein encoded by the FAD2-1A gene has a sequence identity of at least 70%, at least 75%, at least 80%, at least 85%, at least 90%, at least 91%, at least 92%, at least 93%, at least 94%, at least 95%, at least 96%, at least 97%, at least 98%, or at least 99% with SEQ ID No. 1; the amino acid sequence of the protein encoded by the FAD2-1B gene has a sequence identity of at least 70%, at least 75%, at least 80%, at least 85%, at least 90%, at least 91%, at least 92%, at least 93%, at least 94%, at least 95%, at least 96%, at least 97%, at least 98%, or at least 99% with SEQ ID No. 2; the amino acid sequence of the protein encoded by the Lox1 gene has a sequence identity of at least 70%, at least 75%, at least 80%, at least 85%, at least 90%, at least 91%, at least 92%, at least 93%, at least 94%, at least 95%, at least 96%, at least 97%, at least 98%, or at least 99% with SEQ ID No. 3; the amino acid sequence of the protein encoded by the Lox2 gene has a sequence identity of at least 70%, at least 75%, at least 80%, at least 85%, at least 90%, at least 91%, at least 92%, at least 93%, at least 94%, at least 95%, at least 96%, at least 97%, at least 98%, or at least 99% with SEQ ID No. 4; and the amino acid sequence of the protein encoded by the Lox3 gene has a sequence identity of at least 70%, at least 75%, at least 80%, at least 85%, at least 90%, at least 91%, at least 92%, at least 93%, at least 94%, at least 95%, at least 96%, at least 97%, at least 98%, or at least 99% with SEQ ID No. 5.

In another preferred embodiment, the lipoxygenase and fatty acid desaturase genes are derived from one or more plants selected from the group consisting of *Poaceae, Fabaceae, Chenopodiaceae,* and *Brassicaceae* plants.

In another preferred embodiment, the lipoxygenase and fatty acid desaturase genes are derived from one or more plants selected from the group consisting of *Arabidopsis thaliana,* rice, tobacco, corn, sorghum, barley, wheat, millet, soybean, tomato, potato, quinoa, lettuce, rape, *Brassica rapa,* spinach, *Beta vulgaris,* and strawberry.

In another preferred embodiment, the lipoxygenase and fatty acid desaturase genes are derived from soybean.

In another preferred embodiment, the beany-flavor compound is selected from the group consisting of hexanal, 2-hexenal, 1-hexanol, 1-octen-3-ol, trans-2-octenal, nonanal, trans-2-nonenal, isovaleraldehyde, hexanone, isoamyl alcohol, heptanol, acetic acid, propionic acid, dimethylamine, benzene, benzaldehyde, fumaric acid, salicylic acid, ferulic acid, or a combination thereof.

In another preferred embodiment, the beany-flavor compound is selected from the group consisting of hexanal, 2-hexenal, 1-hexanol, 1-octen-3-ol, trans-2-octenal, nonanal, trans-2-nonenal, or a combination thereof.

In an embodiment, the reducing or inhibiting expression levels and/or activities of lipoxygenase and fatty acid desaturase genes or proteins encoded thereby in the plant is achieved through a mutation to the lipoxygenase and fatty acid desaturase genes. Preferably, the mutation leads to a complete or partial loss of functions of the lipoxygenase and fatty acid desaturase genes. Preferably, the mutation leads to a complete loss of functions of the lipoxygenase and fatty acid desaturase genes. Preferably, the mutation refers to a deletion of partial bases in nucleotide sequences of the lipoxygenase and fatty acid desaturase genes relative to a respective sequence shown in any of SEQ ID NO. 6-10. Preferably, the mutation refers to an insertion of one or more bases in nucleotide sequences of the lipoxygenase and fatty acid desaturase genes relative to a respective sequence shown in any one of SEQ ID NO. 6-10.

In another preferred embodiment, the method includes administering an inhibitor for the lipoxygenase and fatty acid desaturase genes or the proteins encoded thereby.

In another preferred embodiment, the reducing or inhibiting means that expression levels E1 of the lipoxygenase and fatty acid desaturase genes or the proteins encoded thereby in the plant are 0% to 80%, preferably 0% to 60%, more preferably 0% to 40%, and even more preferably 0% to 30% of expression levels E0 of the lipoxygenase and fatty acid desaturase genes or the proteins encoded thereby in a wild-type (WT) plant.

In another preferred embodiment, the reducing or inhibiting expression levels and/or activities of lipoxygenase and fatty acid desaturase genes or proteins encoded thereby is achieved by a technology selected from the group consisting of gene mutation, gene knockout, gene disruption, an RNA interference technology, a gene editing technology, introduction of an inhibitor for the genes or the proteins, or a combination thereof.

In another preferred embodiment, the gene mutation is achieved through one or more of the following approaches: natural variation, physical mutagenesis (such as ultraviolet-induced mutagenesis, X-ray-induced mutagenesis, or Y-ray-induced mutagenesis), chemical mutagenesis (such as nitrous acid, hydroxylamine, ethyl methanesulfonate (EMS), and nitrosoguanidine), biological mutagenesis (such as virus or bacterium-mediated mutagenesis), gene editing, or biosynthesis.

In another preferred embodiment, a mutation region includes an exon and/or intron region.

In another preferred embodiment, the inhibitor is selected from the group consisting of an antisense nucleic acid, an antibody, a small-molecule compound, a Crispr reagent, a small-molecule ligand, or a combination thereof.

In another preferred embodiment, the gene editing technology is selected from the group consisting of a clustered regularly interspaced short palindromic repeat (CRISPR) technology, a transcription activator-like effector nuclease (TALEN) technology, a zinc finger nuclease (ZFN) technology, or a combination thereof.

In another preferred embodiment, the method includes the following steps:
(i) providing a plant or a plant cell; and
(ii) introducing an inhibitor for the lipoxygenase and fatty acid desaturase genes or the proteins encoded thereby into the plant or the plant cell to produce a modified plant or plant cell.

In another preferred embodiment, the method includes the following steps:
(i) providing a plant or a plant cell; and
(ii) introducing guide RNA (gRNA) targeting the lipoxygenase and fatty acid desaturase genes and a corresponding Cas protein into the plant or the plant cell. In a preferred embodiment, an expression vector carrying the gRNA and the Cas protein is introduced into the plant or the plant cell.

In another preferred embodiment, the lipoxygenase and fatty acid desaturase genes include a cDNA sequence, a CDS sequence, a genomic sequence, or a combination thereof.

In another preferred embodiment, the reducing or inhibiting expression levels and/or activities of lipoxygenase and fatty acid desaturase genes or proteins encoded thereby is achieved through a mutation to the lipoxygenase and fatty acid desaturase genes.

In another preferred embodiment, the reducing or inhibiting expression levels and/or activities of lipoxygenase and fatty acid desaturase genes or proteins encoded thereby is achieved through a mutation to the FAD2-1A gene, the FAD2-1B gene, the Lox1 gene, the Lox2 gene, and the Lox3 gene simultaneously.

In another preferred embodiment, the mutation includes an insertion mutation, a deletion mutation, a frameshift mutation, and a substitution mutation.

In another preferred embodiment, the method further includes a step of testing a reduction level for the content of the beany-flavor compound in the plant.

In another preferred embodiment, the content of the beany-flavor compound in the plant is 0 or 0.001 µg/g to 20 µg/g, preferably 0.01 µg/g to 5 µg/g, more preferably 0.001 µg/g to 1 µg/g, even more preferably 0.001 µg/g to 0.5 µg/g, and most preferably 0.001 µg/g to 0.1 µg/g.

In another preferred embodiment, the plant includes a monocotyledonous plant and a dicotyledonous plant.

In another preferred embodiment, the plant is one or more plants selected from the group consisting of *Fabaceae, Brassicaceae, Poaceae, Solanaceae, Cucurbitaceae, Chenopodiaceae, Polygonaceae, Pedaliaceae, Asteraceae, Malvaceae, Rosaceae, Pedaliaceae, Convolvulaceae, Dioscoreaceae, Apiaceae, Liliaceae, Zingiberaceae,* and *Arecaceae* plants.

In another preferred embodiment, the plant is one or more plants selected from the group consisting of soybean, *Arabidopsis thaliana,* rice, tobacco, tomato, potato, corn, cotton, *Medicago,* sorghum, barley, wheat, millet, sweet potato, quinoa, lettuce, rape, *Brassica rapa,* spinach, *Beta vulgaris,* peanut, watermelon, *Brassica rapa,* strawberry, cucumber, coconut, or a combination thereof.

In another preferred embodiment, the plant is selected from soybean, *Arabidopsis thaliana,* rice, tobacco, tomato, potato, corn, cotton, peanut, sorghum, cucumber, and coconut.

In another preferred embodiment, the plant is selected from soybean.

A second aspect of the present disclosure provides a composition for reducing a content of a beany-flavor compound in a plant or reducing a beany flavor of the plant, including:
(a) an inhibitor for lipoxygenase and fatty acid desaturase genes or proteins encoded thereby in the plant; and optionally,
(b) an agriculturally-acceptable carrier.

In another preferred embodiment, the lipoxygenase gene is selected from any one or more of a Lox1 gene, a Lox2 gene, or a Lox3 gene.

In a preferred embodiment, the inhibitor can reduce or inhibit expression levels and/or activities of the Lox1 gene, the Lox2 gene, and the Lox3 gene or proteins encoded thereby.

In another preferred embodiment, the fatty acid desaturase gene is selected from an FAD2-1A gene and/or an FAD2-1B gene.

In a preferred embodiment, the inhibitor can reduce or inhibit expression levels and/or activities of the FAD2-1A gene and the FAD2-1B gene or proteins encoded thereby.

In another preferred embodiment, an amino acid sequence of a protein encoded by the FAD2-1A gene has a sequence identity of at least 70%, at least 75%, at least 80%, at least 85%, at least 90%, at least 91%, at least 92%, at least 93%, at least 94%, at least 95%, at least 96%, at least 97%, at least 98%, or at least 99% with SEQ ID No. 1; an amino acid sequence of a protein encoded by the FAD2-1B gene has a sequence identity of at least 70%, at least 75%, at least 80%, at least 85%, at least 90%, at least 91%, at least 92%, at least 93%, at least 94%, at least 95%, at least 96%, at least 97%, at least 98%, or at least 99% with SEQ ID No. 2; an amino acid sequence of a protein encoded by the Lox1 gene has a sequence identity of at least 70%, at least 75%, at least 80%, at least 85%, at least 90%, at least 91%, at least 92%, at least 93%, at least 94%, at least 95%, at least 96%, at least 97%, at least 98%, or at least 99% with SEQ ID No. 3; an amino acid sequence of a protein encoded by the Lox2 gene has a sequence identity of at least 70%, at least 75%, at least 80%, at least 85%, at least 90%, at least 91%, at least 92%, at least 93%, at least 94%, at least 95%, at least 96%, at least 97%, at least 98%, or at least 99% with SEQ ID No. 4; and an amino acid sequence of a protein encoded by the Lox3 gene has a sequence identity of at least 70%, at least 75%, at least 80%, at least 85%, at least 90%, at least 91%, at least 92%, at least 93%, at least 94%, at least 95%, at least 96%, at least 97%, at least 98%, or at least 99% with SEQ ID No. 5.

In another preferred embodiment, the lipoxygenase and fatty acid desaturase genes are derived from one or more plants selected from the group consisting of *Poaceae, Fabaceae, Chenopodiaceae,* and *Brassicaceae* plants.

In another preferred embodiment, the lipoxygenase and fatty acid desaturase genes are derived from one or more plants selected from the group consisting of *Arabidopsis thaliana,* rice, tobacco, corn, sorghum, barley, wheat, millet, soybean, tomato, potato, quinoa, lettuce, rape, *Brassica rapa,* spinach, *Beta vulgaris,* and strawberry.

In another preferred embodiment, the lipoxygenase and fatty acid desaturase genes are derived from soybean.

In another preferred embodiment, the composition includes an agricultural composition.

In another preferred embodiment, the inhibitor includes an agricultural inhibitor.

In another preferred embodiment, a dosage form of the composition is selected from the group consisting of a solution, an emulsion, a suspension, a powder, a foam, a paste, a granule, an aerosol, or a combination thereof.

In another preferred embodiment, the inhibitor is selected from the group consisting of a gene editing reagent, an antisense nucleic acid, an antibody, a small-molecule compound, a Crispr reagent, a small-molecule ligand, or a combination thereof.

In another preferred embodiment, the antisense nucleic acid is selected from the group consisting of antisense RNA, antisense DNA, interfering RNA, a ribozyme, or a combination thereof.

In another preferred embodiment, the interfering RNA is selected from the group consisting of siRNA, shRNA, RNAi, miRNA, dsRNA, hpRNA, ihpRNA, or a combination thereof.

In another preferred embodiment, the composition further includes other substances for reducing the beany flavor of the plant.

In another preferred embodiment, the reducing a beany flavor of a plant includes lowering a content of a beany-flavor compound in the plant.

In another preferred embodiment, the beany-flavor compound is selected from the group consisting of hexanal, 2-hexenal, 1-hexanol, 1-octen-3-ol, trans-2-octenal, nonanal, trans-2-nonenal, isovaleraldehyde, hexanone, isoamyl alcohol, heptanol, acetic acid, propionic acid, dimethylamine, benzene, benzaldehyde, fumaric acid, salicylic acid, ferulic acid, or a combination thereof.

In another preferred embodiment, the beany-flavor compound is selected from the group consisting of hexanal, 2-hexenal, 1-hexanol, 1-octen-3-ol, trans-2-octenal, nonanal, trans-2-nonenal, or a combination thereof.

In another preferred embodiment, the plant includes a monocotyledonous plant and a dicotyledonous plant.

In another preferred embodiment, the plant is one or more plants selected from the group consisting of *Fabaceae, Brassicaceae, Poaceae, Solanaceae, Cucurbitaceae, Chenopodiaceae, Polygonaceae, Pedaliaceae, Asteraceae, Malvaceae, Rosaceae, Pedaliaceae, Convolvulaceae, Dioscoreaceae, Apiaceae, Liliaceae, Zingiberaceae,* and *Arecaceae* plants.

In another preferred embodiment, the plant is one or more plants selected from the group consisting of soybean, *Arabidopsis thaliana,* rice, tobacco, tomato, potato, corn, cotton, *Medicago,* sorghum, barley, wheat, millet, sweet potato, quinoa, lettuce, rape, *Brassica rapa,* spinach, *Beta vulgaris,* peanut, watermelon, *Brassica rapa,* strawberry, cucumber, coconut, or a combination thereof.

In another preferred embodiment, the plant is selected from soybean, *Arabidopsis thaliana,* rice, tobacco, tomato, potato, corn, cotton, peanut, sorghum, cucumber, and coconut.

In another preferred embodiment, the plant is selected from soybean.

A third aspect of the present disclosure provides a use of the composition described in the second aspect of the present disclosure for reducing a content of a beany-flavor compound in a plant or reducing a beany flavor of the plant.

In another preferred embodiment, the use of the composition refers to a use in preparation of a reagent or kit for reducing a content of a beany-flavor compound in a plant or reducing a beany flavor of the plant.

A fourth aspect of the present disclosure provides a method for preparing a plant cell, a plant seed, a plant tissue, a plant part, or a plant with a reduced content of a beany-flavor compound or a reduced beany flavor, including the following step:
reducing or inhibiting expression levels and/or activities of lipoxygenase and fatty acid desaturase genes or proteins encoded thereby in a plant cell, a plant seed, a plant tissue, a plant part, or a plant to produce the plant cell, the plant seed, the plant tissue, the plant part, or the plant with the reduced content of the beany-flavor compound or the reduced beany flavor.

In another preferred embodiment, the lipoxygenase gene is selected from any one or more of a Lox1 gene, a Lox2 gene, or a Lox3 gene.

In a preferred embodiment, the method includes reducing or inhibiting expression levels and/or activities of a Lox1 gene, a Lox2 gene, and a Lox3 gene or proteins encoded thereby.

In another preferred embodiment, the fatty acid desaturase gene is selected from an FAD2-1A gene and/or an FAD2-1B gene.

In a preferred embodiment, the method includes reducing or inhibiting expression levels and/or activities of an FAD2-1A gene and an FAD2-1B gene or proteins encoded thereby.

In another preferred embodiment, an amino acid sequence of a protein encoded by the FAD2-1A gene has a sequence identity of at least 70%, at least 75%, at least 80%, at least 85%, at least 90%, at least 91%, at least 92%, at least 93%, at least 94%, at least 95%, at least 96%, at least 97%, at least 98%, or at least 99% with SEQ ID No. 1; an amino acid sequence of a protein encoded by the FAD2-1B gene has a sequence identity of at least 70%, at least 75%, at least 80%, at least 85%, at least 90%, at least 91%, at least 92%, at least 93%, at least 94%, at least 95%, at least 96%, at least 97%, at least 98%, or at least 99% with SEQ ID No. 2; an amino acid sequence of a protein encoded by the Lox1 gene has a sequence identity of at least 70%, at least 75%, at least 80%, at least 85%, at least 90%, at least 91%, at least 92%, at least 93%, at least 94%, at least 95%, at least 96%, at least 97%, at least 98%, or at least 99% with SEQ ID No. 3; an amino acid sequence of a protein encoded by the Lox2 gene has a sequence identity of at least 70%, at least 75%, at least 80%, at least 85%, at least 90%, at least 91%, at least 92%, at least 93%, at least 94%, at least 95%, at least 96%, at least 97%, at least 98%, or at least 99% with SEQ ID No. 4; and an amino acid sequence of a protein encoded by the Lox3 gene has a sequence identity of at least 70%, at least 75%, at least 80%, at least 85%, at least 90%, at least 91%, at least 92%, at least 93%, at least 94%, at least 95%, at least 96%, at least 97%, at least 98%, or at least 99% with SEQ ID No. 5.

In another preferred embodiment, the lipoxygenase and fatty acid desaturase genes are derived from one or more plants selected from the group consisting of *Poaceae, Fabaceae, Chenopodiaceae,* and *Brassicaceae* plants.

In another preferred embodiment, the lipoxygenase and fatty acid desaturase genes are derived from one or more plants selected from the group consisting of *Arabidopsis thaliana,* rice, tobacco, corn, sorghum, barley, wheat, millet, soybean, tomato, potato, quinoa, lettuce, rape, *Brassica rapa,* spinach, *Beta vulgaris,* and strawberry.

In another preferred embodiment, the lipoxygenase and fatty acid desaturase genes are derived from soybean.

In another preferred embodiment, the beany-flavor compound is selected from the group consisting of hexanal, 2-hexenal, 1-hexanol, 1-octen-3-ol, trans-2-octenal, nonanal, trans-2-nonenal, isovaleraldehyde, hexanone, isoamyl alcohol, heptanol, acetic acid, propionic acid, dimethylamine, benzene, benzaldehyde, fumaric acid, salicylic acid, ferulic acid, or a combination thereof.

In another preferred embodiment, the beany-flavor compound is selected from the group consisting of hexanal, 2-hexenal, 1-hexanol, 1-octen-3-ol, trans-2-octenal, nonanal, trans-2-nonenal, or a combination thereof.

In another preferred embodiment, the reducing or inhibiting expression levels and/or activities of lipoxygenase and fatty acid desaturase genes or proteins encoded thereby is achieved by a technology selected from the group consisting of gene mutation, gene knockout, gene disruption, an RNA interference technology, a gene editing technology, introduction of an inhibitor for the genes or the proteins, or a combination thereof.

In another preferred embodiment, the plant includes a monocotyledonous plant and a dicotyledonous plant.

In another preferred embodiment, the plant is one or more plants selected from the group consisting of *Fabaceae, Brassicaceae, Poaceae, Solanaceae, Cucurbitaceae, Chenopodiaceae, Polygonaceae, Pedaliaceae, Asteraceae, Malvaceae, Rosaceae, Pedaliaceae, Convolvulaceae, Dioscoreaceae, Apiaceae, Liliaceae, Zingiberaceae,* and *Arecaceae* plants.

In another preferred embodiment, the plant is one or more plants selected from the group consisting of soybean, *Arabidopsis thaliana,* rice, tobacco, tomato, potato, corn, cotton, *Medicago,* sorghum, barley, wheat, millet, sweet potato, quinoa, lettuce, rape, *Brassica rapa,* spinach, *Beta vulgaris,* peanut, watermelon, *Brassica rapa,* strawberry, cucumber, coconut, or a combination thereof.

In another preferred embodiment, the plant is selected from soybean, *Arabidopsis thaliana,* rice, tobacco, tomato, potato, corn, cotton, peanut, sorghum, cucumber, and coconut.

In another preferred embodiment, the plant is selected from soybean.

A fifth aspect of the present disclosure provides a method for preparing a plant with a reduced content of a beany-flavor compound or a reduced beany flavor, including the following step:
regenerating a plant from the plant cell, the plant seed, the plant tissue, or the plant part with the reduced content of the beany-flavor compound or the reduced beany flavor prepared by the method described in the fourth aspect of the present disclosure, so as to obtain the plant with the reduced content of the beany-flavor compound or the reduced beany flavor.

In another preferred embodiment, the method further includes a step of harvesting a plant seed from the plant with the reduced content of the beany-flavor compound or the reduced beany flavor.

A sixth aspect of the present disclosure provides a plant cell, a plant seed, a plant tissue, a plant part, or a plant with a reduced content of a beany-flavor compound or a reduced beany flavor that is prepared by the method described in the fourth or fifth aspect of the present disclosure.

In another preferred embodiment, the beany-flavor compound is selected from the group consisting of hexanal, 2-hexenal, 1-hexanol, 1-octen-3-ol, trans-2-octenal, nonanal, trans-2-nonenal, isovaleraldehyde, hexanone, isoamyl alcohol, heptanol, acetic acid, propionic acid, dimethylamine, benzene, benzaldehyde, fumaric acid, salicylic acid, ferulic acid, or a combination thereof.

In another preferred embodiment, the beany-flavor compound is selected from the group consisting of hexanal, 2-hexenal, 1-hexanol, 1-octen-3-ol, trans-2-octenal, nonanal, trans-2-nonenal, or a combination thereof.

A seventh aspect of the present disclosure provides a method for improving a plant, including the following steps:
(a) providing a plant cell, a plant tissue, or a plant part, and introducing an inhibitor for lipoxygenase and fatty acid desaturase genes or proteins encoded thereby into the plant cell, the plant tissue, or the plant part; and
(b) regenerating a plant from a plant cell, a plant tissue, or a plant part obtained in the step (a).

In another preferred embodiment, the lipoxygenase gene is selected from any one or more of a Lox1 gene, a Lox2 gene, or a Lox3 gene.

In another preferred embodiment, the fatty acid desaturase gene is selected from an FAD2-1A gene and/or an FAD2-1B gene.

In another preferred embodiment, an amino acid sequence of a protein encoded by the FAD2-1A gene has a sequence identity of at least 70%, at least 75%, at least 80%, at least 85%, at least 90%, at least 91%, at least 92%, at least 93%, at least 94%, at least 95%, at least 96%, at least 97%, at least 98%, or at least 99% with SEQ ID No. 1; an amino acid sequence of a protein encoded by the FAD2-1B gene has a sequence identity of at least 70%, at least 75%, at least 80%, at least 85%, at least 90%, at least 91%, at least 92%, at least 93%, at least 94%, at least 95%, at least 96%, at least 97%, at least 98%, or at least 99% with SEQ ID No. 2; an amino acid sequence of a protein encoded by the Lox1 gene has a sequence identity of at least 70%, at least 75%, at least 80%, at least 85%, at least 90%, at least 91%, at least 92%, at least 93%, at least 94%, at least 95%, at least 96%, at least 97%, at least 98%, or at least 99% with SEQ ID No. 3; an amino acid sequence of a protein encoded by the Lox2 gene has a sequence identity of at least 70%, at least 75%, at least 80%, at least 85%, at least 90%, at least 91%, at least 92%, at least 93%, at least 94%, at least 95%, at least 96%, at least 97%, at least 98%, or at least 99% with SEQ ID No. 4; and an amino acid sequence of a protein encoded by the Lox3 gene has a sequence identity of at least 70%, at least 75%, at least 80%, at least 85%, at least 90%, at least 91%, at least 92%, at least 93%, at least 94%, at least 95%, at least 96%, at least 97%, at least 98%, or at least 99% with SEQ ID No. 5.

In another preferred embodiment, the lipoxygenase and fatty acid desaturase genes are derived from one or more plants selected from the group consisting of *Poaceae, Fabaceae, Chenopodiaceae,* and *Brassicaceae* plants.

In another preferred embodiment, the lipoxygenase and fatty acid desaturase genes are derived from one or more plants selected from the group consisting of *Arabidopsis thaliana,* rice, tobacco, corn, sorghum, barley, wheat, millet, soybean, tomato, potato, quinoa, lettuce, rape, *Brassica rapa,* spinach, *Beta vulgaris,* and strawberry.

In another preferred embodiment, the lipoxygenase and fatty acid desaturase genes are derived from soybean.

In another preferred embodiment, in the step (a), the plant cell, the plant tissue, or the plant part is modified by a gene editing technology to reduce expression levels or activities of the lipoxygenase and fatty acid desaturase genes or the proteins encoded thereby in the plant cell, the plant tissue, or the plant part.

In another preferred embodiment, the gene editing technology is selected from the group consisting of a CRISPR gene editing system, error-prone polymerase chain reaction (PCR), gene recombination, TALEN, and ZFN.

In another preferred embodiment, the method is provided to reduce a content of a beany-flavor compound in a plant.

In another preferred embodiment, the method is provided to reduce a beany flavor of a plant.

In another preferred embodiment, the beany-flavor compound is selected from the group consisting of hexanal, 2-hexenal, 1-hexanol, 1-octen-3-ol, trans-2-octenal, nonanal, trans-2-nonenal, isovaleraldehyde, hexanone, isoamyl alcohol, heptanol, acetic acid, propionic acid, dimethylamine, benzene, benzaldehyde, fumaric acid, salicylic acid, ferulic acid, or a combination thereof.

In another preferred embodiment, the beany-flavor compound is selected from the group consisting of hexanal, 2-hexenal, 1-hexanol, 1-octen-3-ol, trans-2-octenal, nonanal, trans-2-nonenal, or a combination thereof.

In another preferred embodiment, the method further includes a step of testing a reduction level for a content of a beany-flavor compound in the plant cell, the plant tissue, the plant part, or the plant.

In another preferred embodiment, the plant includes a monocotyledonous plant and a dicotyledonous plant.

In another preferred embodiment, the plant is one or more plants selected from the group consisting of *Fabaceae, Brassicaceae, Poaceae, Solanaceae, Cucurbitaceae, Chenopodiaceae, Polygonaceae, Pedaliaceae, Asteraceae, Malvaceae, Rosaceae, Pedaliaceae, Convolvulaceae, Dioscoreaceae, Apiaceae, Liliaceae, Zingiberaceae,* and *Arecaceae* plants.

In another preferred embodiment, the plant is one or more plants selected from the group consisting of soybean, *Arabidopsis thaliana,* rice, tobacco, tomato, potato, corn, cotton, *Medicago,* sorghum, barley, wheat, millet, sweet potato, quinoa, lettuce, rape, *Brassica rapa,* spinach, *Beta vulgaris,* peanut, watermelon, *Brassica rapa,* strawberry, cucumber, coconut, or a combination thereof.

In another preferred embodiment, the plant is selected from soybean, *Arabidopsis thaliana,* rice, tobacco, tomato, potato, corn, cotton, peanut, sorghum, cucumber, and coconut.

In another preferred embodiment, the plant is selected from soybean.

An eighth aspect of the present disclosure provides a genetically engineered plant prepared by the method described in the seventh aspect of the present disclosure.

A ninth aspect of the present disclosure provides a method for screening or identifying a plant with a low beany flavor or a plant with no beany flavor, including a step of detecting expression levels of lipoxygenase and fatty acid desaturase genes or proteins encoded thereby in the plant.

In another preferred embodiment, the lipoxygenase gene is selected from any one or more of a Lox1 gene, a Lox2 gene, or a Lox3 gene.

In another preferred embodiment, the fatty acid desaturase gene is selected from an FAD2-1A gene and/or an FAD2-1B gene.

In another preferred embodiment, an amino acid sequence of a protein encoded by the FAD2-1A gene has a sequence identity of at least 70%, at least 75%, at least 80%, at least 85%, at least 90%, at least 91%, at least 92%, at least 93%, at least 94%, at least 95%, at least 96%, at least 97%, at least 98%, or at least 99% with SEQ ID No. 1; an amino acid sequence of a protein encoded by the FAD2-1B gene has a sequence identity of at least 70%, at least 75%, at least 80%, at least 85%, at least 90%, at least 91%, at least 92%, at least 93%, at least 94%, at least 95%, at least 96%, at least 97%, at least 98%, or at least 99% with SEQ ID No. 2; an amino acid sequence of a protein encoded by the Lox1 gene has a sequence identity of at least 70%, at least 75%, at least 80%, at least 85%, at least 90%, at least 91%, at least 92%, at least 93%, at least 94%, at least 95%, at least 96%, at least 97%, at least 98%, or at least 99% with SEQ ID No. 3; an amino acid sequence of a protein encoded by the Lox2 gene has a sequence identity of at least 70%, at least 75%, at least 80%, at least 85%, at least 90%, at least 91%, at least 92%, at least 93%, at least 94%, at least 95%, at least 96%, at least 97%, at least 98%, or at least 99% with SEQ ID No. 4; and an amino acid sequence of a protein encoded by the Lox3 gene has a sequence identity of at least 70%, at least 75%, at least 80%, at least 85%, at least 90%, at least 91%, at least 92%, at least 93%, at least 94%, at least 95%, at least 96%, at least 97%, at least 98%, or at least 99% with SEQ ID No. 5.

In another preferred embodiment, the lipoxygenase and fatty acid desaturase genes are derived from one or more plants selected from the group consisting of *Poaceae, Fabaceae, Chenopodiaceae,* and *Brassicaceae* plants.

In another preferred embodiment, the lipoxygenase and fatty acid desaturase genes are derived from one or more plants selected from the group consisting of *Arabidopsis thaliana,* rice, tobacco, corn, sorghum, barley, wheat, millet, soybean, tomato, potato, quinoa, lettuce, rape, *Brassica rapa,* spinach, *Beta vulgaris,* and strawberry.

In another preferred embodiment, the lipoxygenase and fatty acid desaturase genes are derived from soybean.

In another preferred embodiment, the low beany flavor or the no beany flavor refers to a low content of a beany-flavor compound.

In another preferred embodiment, the beany-flavor compound is selected from the group consisting of hexanal, 2-hexenal, 1-hexanol, 1-octen-3-ol, trans-2-octenal, nonanal, trans-2-nonenal, isovaleraldehyde, hexanone, isoamyl alcohol, heptanol, acetic acid, propionic acid, dimethylamine, benzene, benzaldehyde, fumaric acid, salicylic acid, ferulic acid, or a combination thereof.

In another preferred embodiment, the beany-flavor compound is selected from the group consisting of hexanal, 2-hexenal, 1-hexanol, 1-octen-3-ol, trans-2-octenal, nonanal, trans-2-nonenal, or a combination thereof.

In another preferred embodiment, the plant includes a monocotyledonous plant and a dicotyledonous plant.

In another preferred embodiment, the plant is one or more plants selected from the group consisting of *Fabaceae, Brassicaceae, Poaceae, Solanaceae, Cucurbitaceae, Chenopodiaceae, Polygonaceae, Pedaliaceae, Asteraceae, Malvaceae, Rosaceae, Pedaliaceae, Convolvulaceae, Dioscoreaceae, Apiaceae, Liliaceae, Zingiberaceae,* and *Arecaceae* plants.

In another preferred embodiment, the plant is one or more plants selected from the group consisting of soybean, *Arabidopsis thaliana,* rice, tobacco, tomato, potato, corn, cotton, *Medicago,* sorghum, barley, wheat, millet, sweet potato, quinoa, lettuce, rape, *Brassica rapa,* spinach, *Beta vulgaris,* peanut, watermelon, *Brassica rapa,* strawberry, cucumber, coconut, or a combination thereof.

In another preferred embodiment, the plant is selected from soybean, *Arabidopsis thaliana,* rice, tobacco, tomato, potato, corn, cotton, peanut, sorghum, cucumber, and coconut.

In another preferred embodiment, the plant is selected from soybean.

In another preferred embodiment, a part for detecting the plant includes a callus, a fruit, a seed, a flower, a stem, a leaf, a spike, and a root of the plant.

A tenth aspect of the present disclosure provides a method for preparing a soybean seed with a low beany flavor or no beany flavor, including a step of preparing the soybean seed with the low beany flavor or no beany flavor using the plant prepared by the method described above.

An eleventh aspect of the present disclosure also provides a soybean seed with a low beany flavor or no beany flavor prepared by the method for preparing a soybean seed with a low beany flavor or no beany flavor described above.

In another preferred embodiment, a content of a beany-flavor compound in the soybean seed with the low beany flavor or no beany flavor is reduced by at least 5%, at least 10%, at least 20%, at least 50%, at least 60%, at least 70%, at least 80%, at least 90%, at least 95%, or at least 98% compared with a WT soybean seed.

In another preferred embodiment, the beany-flavor compound is selected from the group consisting of hexanal, 2-hexenal, 1-hexanol, 1-octen-3-ol, trans-2-octenal, nonanal, trans-2-nonenal, isovaleraldehyde, hexanone, isoamyl alcohol, heptanol, acetic acid, propionic acid, dimethylamine, benzene, benzaldehyde, fumaric acid, salicylic acid, ferulic acid, or a combination thereof.

In another preferred embodiment, the beany-flavor compound is selected from the group consisting of hexanal, 2-hexenal, 1-hexanol, 1-octen-3-ol, trans-2-octenal, nonanal, trans-2-nonenal, or a combination thereof.

A twelfth aspect of the present disclosure further provides a method for preparing a soybean plant, including a step of allowing hybridization between a soybean seed with a low beany flavor or no beany flavor or a soybean plant with a low beany flavor or no beany flavor and another soybean variety to produce a soybean plant.

It should be understood that, within the scope of the present disclosure, the technical features mentioned above and the specific technical features described in detail below (such as in embodiments) can be combined with each other to constitute new or preferred technical solutions, which are not repeated one by one here for conciseness.

### Specific Implementations

Through the extensive and in-depth research, namely, the investigation and screening of numerous plant trait loci, the inventors have unexpectedly discovered for the first time that the simultaneous inhibition on the expression of lipoxygenase and fatty acid desaturase genes or proteins encoded thereby can significantly reduce the content of a beany-flavor compound in a plant. Based on these findings, the inventors have accomplished the present disclosure.

Unless otherwise defined, the technical and scientific terms used herein have the same meanings as commonly understood by those of ordinary skill in the art.

As used herein, the term "beany flavor" includes a beany odor, an undesirable odor, a fishy smell, a pungent odor, an unpleasant smell, etc. The beany-flavor compound includes, but is not limited to, hexanal, 2-hexenal, 1-hexanol, 1-octen-3-ol, trans-2-octenal, nonanal, trans-2-nonenal, isovaleraldehyde, hexanone, isoamyl alcohol, heptanol, acetic acid, propionic acid, dimethylamine, benzene, benzaldehyde, fumaric acid, salicylic acid, and ferulic acid.

As used herein, the terms "polynucleotide", "nucleotide sequence", "nucleic acid sequence", "nucleic acid molecule", and "nucleic acid" can be used interchangeably, and include DNA, RNA, or a hybrid thereof, which may be double-stranded or single-stranded.

As used herein, the term "Crispr reagent" refers to a combination of effective components for achieving a gene editing effect. The Crispr reagent includes gRNA or a coding sequence thereof and a Ca protein or a coding sequence thereof, and can further include a vector and elements favorable for homologous recombination or gene expression.

The term "homology" or "identity" refers to the sequence matching between two polypeptides or between two nucleic acids. When specified positions in two sequences to be compared are occupied by the same base or amino acid monomer subunit (for example, a specified position in each of two DNA molecules is occupied by adenine, or a specified position in each of two polypeptides is occupied by lysine), the two sequences are identical at the position. Generally, the comparison is conducted by aligning two sequences to produce the maximum identity. An alignment method is a conventional technique well known to those skilled in the art, such as the basic local alignment search tool (BLAST) algorithm.

The term "genetic engineering" refers to a technology in which nucleotides controlling biological genetic information are modified and utilized through artificial intervention to produce novel genetic traits, varieties, or products. The genetic engineering includes all genetic modification technologies disclosed in the art, such as gene mutagenesis, transgenic technique, or gene editing. A method for the gene mutagenesis includes, but is not limited to, physical mutagenesis (such as ultraviolet-induced mutagenesis), chemical mutagenesis (such as acridine dyes), and biological mutagenesis (such as virus or bacteriophage-mediated mutagenesis).

A specific amino acid position (number) in the protein of the present disclosure is determined by aligning an amino acid sequence of the target protein with any one of SEQ ID No. 1-5 using a standard sequence alignment tool. For example, the Smith-Waterman algorithm or the CLUSTALW2 algorithm is used to align two sequences, and the sequences are considered aligned when an alignment score is the highest. The alignment score can be calculated according to the method described in Wilbur, W.J. and Lipman,D.J. (1983) Rapid similarity searches of nucleic acid and protein data banks. Proc. Natl. Acad. Sci. USA, 80: 726-730. In the ClustalW2(1.82) algorithm, default parameters are preferably adopted: protein gap opening penalty = 10.0; protein gap extension penalty = 0.2; protein matrix = Gonnet; protein/DNA end gap = -1; and protein/DNAGAPDIST = 4. The AlignX program (a part of the vectorNTI group) may preferably be used to align an amino acid sequence of the protein of the present disclosure with any one of SEQ ID No. 1-5 under default parameters suitable for multiple alignment (gap opening penalty: 10; and gap extension penalty: 0.05) to determine a position of a specific amino acid in the protein.

The term "encoding" refers to an inherent characteristic of a specific nucleotide sequence in a polynucleotide, such as a gene cDNA or mRNA, which serves as a template for the synthesis of a defined nucleotide sequence (namely, rRNA, tRNA, and mRNA) or a defined amino acid sequence and the synthesis of other polymers and macromolecules in a biological process of a biological characteristic thereof. Therefore, if the transcription and translation of mRNA corresponding to a gene produces a protein in a cell or another biological system, the gene encodes the protein.

The term "amino acid" refers to a carboxylic acid with amino. Various proteins in organisms each are composed of 20 essential amino acids.

The terms "protein", "polypeptide", and "peptide" can be used interchangeably in the present disclosure, and refer to a polymer of amino acid residues, including a polymer in which one or more amino acid residues are a chemical analog of a natural amino acid residue. The protein and polypeptide of the present disclosure can be produced through recombination or chemical synthesis.

In the present disclosure, an amino acid residue can be represented by a single letter or three letters, such as: alanine (Ala, A), valine (Val, V), glycine (Gly, G), leucine (Leu, L), glutamine (Gln, Q), phenylalanine (Phe, F), tryptophan (Trp, W), tyrosine (Tyr, Y), aspartic acid (Asp, D), asparagine (Asn, N), glutamic acid (Glu, E), lysine (Lys, K), methionine (Met, M), serine (Ser, S), threonine (Thr, T), cysteine (Cys, C), proline (Pro, P), isoleucine (Ile, I), histidine (His, H), and arginine (Arg, R).

As used herein, the term "regulatory element", also known as "regulation element", is intended to include a promoter, a terminator sequence, a leader sequence, a polyadenylation sequence, a signal peptide coding region, a marker gene, an enhancer, an internal ribosome entry site (IRES), and other expression control elements (for example, a transcriptional termination signal, such as a polyadenylation signal and a poly-U sequence), and the detailed description can be seen in Goeddel, "GENE EXPRESSION TECHNOLOGY: METHODS IN ENZYMOLOGY" 185, Academic Press, San Diego, California (1990). In some cases, a regulatory element includes sequences that guide the constitutive expression of a nucleotide sequence in many types of host cells and sequences that guide the expression of the nucleotide sequence only in some host cells (such as a tissue-specific regulatory sequence). A tissue-specific promoter can mainly guide the expression in a desired tissue of interest, such as muscles, neurons, bones, skin, blood, specific organs (such as liver and pancreas), or specific cell types (such as lymphocytes). In some cases, a regulatory element can also guide the expression in a time-dependent manner (such as in a cell cycle-dependent or developmental stage-dependent manner), which may be or may not be tissue or cell type-specific. In some cases, the term "regulatory element" covers enhancer elements, such as WPRE; CMV enhancer; R-U5' fragment in LTR of HTLV-I ((Mol. Cell. Biol., Vol 8 (1): 466-472, 1988); SV40 enhancer; and an intron sequence between exons 2 and 3 of rabbit β-globin (Proc. Natl. Acad. Sci. USA., Vol. 78 (3): 1527-31, 1981).

The term "promoter" has the meaning well known to those skilled in the art, and refers to a non-coding nucleotide sequence that is located upstream of a gene and can promote the expression of a downstream gene. A constitutive promoter is a nucleotide sequence that will result in the generation of a gene product in a cell under most or all physiological conditions of the cell after the promoter is operably linked to a polynucleotide encoding or defining the gene product. An inducible promoter is a nucleotide sequence that will cause the generation of a gene product in a cell only when there is an inducer corresponding to the promoter in the cell after the promoter is operably linked to a polynucleotide encoding or defining the gene product. A tissue-specific promoter is a nucleotide sequence that will cause the generation of a gene product in a cell basically only when the cell is a cell of the tissue type corresponding to the promoter after the promoter is operably linked to a polynucleotide encoding or defining the gene product.

The term "nuclear localization signal" or "nuclear localization sequence" (NLS) is an amino acid sequence that tags a protein for import into a nucleus through nuclear transport, that is, a protein with NLS is transported into a nucleus. Typically, NLS includes positively-charged Lys or Arg residues that are exposed on a surface of a protein. Exemplary NLS includes, but is not limited to, SV40 large T antigen, EGL-13, c-Myc, and TUS protein.

The term "operably linked" means that a nucleotide sequence of interest is linked to one or more regulatory elements in a manner allowing the expression of the nucleotide sequence (for example, in an *in vitro* transcription/translation system or in a host cell when the vector is introduced into the host cell).

The term "vector" refers to an element that is allowed to be integrated into a genome of a host cell or to self-replicate within a cell independently of its genome. The vector may include any elements that guarantee the self-replication. The vector usually carries a gene that is not a part of the central metabolism of a cell, and is usually in the form of double-stranded DNA. The selection of a vector generally depends on the compatibility of the vector with a host cell into which the vector is to be introduced. When a vector needs to be adopted, the selection of the vector depends on a method for transforming a host cell well known to those skilled in the art. For example, a plasmid vector can be used.

The vectors applicable in the present disclosure include commercially-available plasmids, including, but not limited to: pBR322 (ATCC37017), pKK223-3 (Pharmacia Fine Chemicals, Uppsala, Sweden), GEM1 (Promega Biotec, Madison, WI, USA), pQE70, pQE60, pQE-9 (Qiagen), pD10, psiX174pBluescriptIIKS, pNH8A, pNH16a, pNH18A, pNH46A (Stratagene), ptrc99a, pKK223-3, pKK233-3, pDR540, pRIT5 (Pharmacia), pKK232-8, pCM7, pSV2CAT, pOG44, pXT1, pSG (Stratagene), pSVK3, pBPV, pMSG, and pSVL (Pharmacia).

The nucleic acid sequence, nucleic acid construct, or expression vector of the present disclosure can be introduced into a host cell through a variety of techniques, including transformation, transfection, transduction, viral infection, gene gun or Ti-plasmid-mediated gene delivery, calcium phosphate transfection, diethylaminoethyl (DEAE)-dextran-mediated transfection, lipofection, electroporation, etc.

The term "plant tissue" or "plant part" includes a plant cell, a protoplast, a plant tissue culture, a plant callus, a plant piece, a plant embryo, a pollen, an ovule, a seed, a leaf, a stem, a flower, a branch, a seedling, a fruit, a kernel, a spike, a root, a root tip, an anther, etc.

The term "plant cell" should be understood as any cell derived from or found in a plant, and can produce, for example, undifferentiated tissues such as calli, differentiated tissues such as embryos, constituent parts of a plant, plants, or seeds.

The term "plant" should be understood as any differentiated multicellular organism capable of conducting photosynthesis, including: any crop plants at a mature or developmental stage, especially monocotyledonous or dicotyledonous plants; vegetable crops including artichoke, turnip cabbage, arugula, leek, asparagus, lettuce (such as head lettuce, leaf lettuce, and romaine lettuce), bok choy, malanga, melons (such as cantaloupe, watermelon, crenshaw melon, honeydew melon, and Cantaloupe), rape crops (such as Brussels sprout, cabbage, cauliflower, broccoli, borecole, kale, Chinese cabbage, and bok choy), cardoon, carrot, napa, okra, onion, celery, parsley, chickpea, parsnip, chicory, pepper, potato, gourd (such as marrow squash, cucumber, zucchini, cushaw, and pumpkin), radish, dried ball onion, rutabaga, purple eggplant (also known as eggplant), salsify, lettuce, shallot, endive, garlic, spinach, green onion, cushaw, greens, *Beta vulgaris* (sugar beets and fodder beets), sweet potato, Swiss chard, wasabi, tomato, turnip, and spices; fruits and/or vine crops such as apple, apricot, cherry, nectarine, peach, pear, plum, prune, cherry, quince, almond, chestnut, hazelnut, pecan, pistachio, walnut, citrus, blueberry, boysenberry, cranberry, currant, loganberry, raspberry, strawberry, blackberry, grape, avocado, banana, kiwi, persimmon, pomegranate, pineapple, tropical fruit, pome, melon, mango, papaya, and lychee; field crops, such as clover, *Medicago,* evening primrose, meadowfoam, corn/maize (forage maize, sweet corn, and popcorn), lupulus, jojoba, peanut, rice, safflower, small grain crops (barley, oat, rye, wheat, etc.), sorghum, tobacco, kapok, *Fabaceae* plants (beans, lentil, pea, and soybean), oil plants (rape, leaf mustard, poppy, olive, sunflower, coconut, castor oil plant, cocoa bean, and groundnut), *Arabidopsis,* fiber plants (cotton, flax, and jute), *Lauraceae* (cinnamon and camphor), or a plant such as coffee, sugar cane, tea, and natural rubber plants; and/or bedding plants such as a flowering plant, cactus, a succulent plant, and/or an ornamental plant, and trees such as forests (broad-leaved and evergreen trees, such as conifers), fruit trees, ornamental trees, nut-bearing trees, shrubs, and other seedlings.

The gene editing technology includes CRISPR technology, TALEN technology, and ZFN technology. The CRISPR technology refers to clustered regularly interspaced short palindromic repeats coming from the immune system of microorganisms. A gene editing tool of CRISPR includes gRNA and Cas protein (such as Cas9, Cpf1, Cas12b, Cas12i, and Cas12j). A gene editing tool of TALEN refers to a restriction enzyme capable of cleaving a specific DNA sequence, which includes a TAL effector DNA binding domain and a DNA cleavage domain. A gene editing tool of ZFN refers to a restriction enzyme capable of cleaving a specific DNA sequence, which includes a zinc-finger DNA binding domain and a DNA cleavage domain. It is well known to those skilled in the art that an intracellular genome can be edited by constructing nucleotides encoding a gene editing tool and other regulatory elements into a suitable vector and then transforming the vector into a cell, where a type of the editing includes gene knockout, insertion, and base editing.

As used herein, the term "gene editing enzyme" refers to a nuclease suitable for CRISPR, TALEN, ZFN, and other editing tools. Preferably, the gene editing enzyme is a CRISPR enzyme, also known as a Cas protein, including, but not limited to: a Cas9 protein, a Cas12 protein, a Cas13 protein, a Cas14 protein, a Csm1 protein, and an FDK1 protein. The Cas proteins refer to a protein family that may exhibit varying structures depending on their origins, such as SpCas9 derived from *Streptococcus pyogenes* and SaCas9 derived from *Staphylococcus aureus.* Cas proteins can also be classified based on structural characteristics (such as domains). For example, the Cas12 family may include Cas12a (also known as Cpf1), Cas12b, Cas12c, Cas12i, etc. The Cas protein may be double-stranded or single-stranded or may have no cleavage activity. The Cas protein of the present disclosure may be WT or a mutant thereof. A mutation type of the mutant may include amino acid substitution, substitution, or deletion. The mutant may or may not change an enzyme cleavage activity of the Cas protein. Those skilled in the art know that a variety of Cas proteins with nucleic acid cleavage activities have been reported in the art. The known proteins or modified variants thereof all can achieve the function of the present disclosure, which is incorporated into the protection scope of the present disclosure by reference.

As used herein, the terms "gRNA", "mature crRNA", and "guide sequence" can be used interchangeably and have the meaning commonly understood by those skilled in the art. Generally, gRNA can include direct repeats and guide sequences, or may be basically composed of or may be composed of direct repeats and guide sequences (also called spacers in the context of endogenous CRISPR systems).

In some cases, the guide sequence can be any polynucleotide sequence that shows sufficient complementarity with a target sequence to hybridize with the target sequence and guide the specific binding of a CRISPR/Cas complex to the target sequence. In an embodiment, under the optimal alignment, a complementarity degree between the guide sequence and a corresponding target sequence is at least 50%, at least 60%, at least 70%, at least 80%, at least 90%, at least 95%, or at least 99%. Determining the optimal alignment is within the competence of those of ordinary skill in the art. For example, there are published and commercially available alignment algorithms and programs, including, but not limited to, Smith-Waterman, Bowtie, Geneious, Biopython, and SeqMan in ClustalW and matlab.

### FAD2 genes

The FAD2 genes are fatty acid desaturase genes, which are key genes controlling the conversion of oleic acid into linoleic acid. There are multiple FAD2 homologous genes in soybean, such as FAD2-1A (Glyma10g42470) and FAD2-1B (Glyma20g24530). A nucleotide sequence of the FAD2-1A gene in soybean is shown in SEQ ID NO.: 6, and a protein sequence encoded thereby is shown in SEQ ID NO.: 1. A nucleotide sequence of the FAD2-1B gene in soybean is shown in SEQ ID NO.: 7, and a protein sequence encoded thereby is shown in SEQ ID NO.: 2.

### Lox genes

The Lox genes are lipoxygenase genes. The corresponding lipoxygenases can catalyze the oxygenation of unsaturated fatty acids to produce volatile substances such as hydrogen peroxide derivatives, and these volatile substances can directly interact with proteins and amino acids in a food to cause a beany flavor and bitterness, which reduce the palatability and nutritional value of the food. There are multiple Lox homologous genes in soybean, such as GmLox1 (Glyma.13g347600), GmLox2 (Glyma.13g347500), and GmLox3 (Glyma.15g026300). A nucleotide sequence of the Lox1 gene in soybean is shown in SEQ ID NO.: 8, and a protein sequence encoded thereby is shown in SEQ ID NO.: 3. A nucleotide sequence of the Lox2 gene in soybean is shown in SEQ ID NO.: 9, and a protein sequence encoded thereby is shown in SEQ ID NO.: 4. A nucleotide sequence of the Lox3 gene in soybean is shown in SEQ ID NO.: 10, and a protein sequence encoded thereby is shown in SEQ ID NO.: 5.

The present disclosure also includes a nucleic acid having a homology of 50% or more (preferably 60% or more, 70% or more, and 80% or more, more preferably 90% or more, even more preferably 95% or more, and most preferably 98% or more, such as 99% or 100%) with the preferred gene sequence (SEQ ID NO.: 6 or 7 or 8 or 9 or 10) of the present disclosure. The "homology" refers to a similarity level (namely, sequence similarity or identity) between two or more nucleic acids based on a percentage of identical positions. In the present disclosure, variants of the genes can be produced through the insertion or deletion of a regulatory region, the random or site-directed mutagenesis, etc.

In the present disclosure, a nucleotide sequence shown in any one of SEQ ID NO.: 6-10 may undergo the substitution, deletion, or addition of one or more nucleotides to produce a derivative sequence corresponding to the nucleotide sequence shown in any one of SEQ ID NO.: 6-10. Due to the codon degeneracy, the derivative sequence may still basically encode a corresponding amino acid sequence shown in one of SEQ ID NO.: 1-5 even when having a relatively-low homology with the nucleotide sequence shown in any one of SEQ ID NO.: 6-10. Further, a derivative sequence produced through the substitution, deletion, or addition of at least one nucleotide in a nucleotide sequence shown in any one of SEQ ID NO.: 6-10 also encompasses a nucleotide sequence that can hybridize with the nucleotide sequence shown in any one of SEQ ID NO.: 6-10 under moderately-stringent conditions and preferably under highly-stringent conditions. These variant forms include (but are not limited to): deletion, insertion, and/or substitution of several (typically 1 to 90, preferably 1 to 60, more preferably 1 to 20, and most preferably 1 to 10) nucleotides, and addition of several (typically 60 or less, preferably 30 or less, more preferably 10 or less, and most preferably 5 or less) nucleotides at a 5' terminus and/or a 3' terminus.

It should be understood that, although the genes provided in the embodiments of the present disclosure are derived from soybean, a gene sequence which is derived from other similar plants and has a specified homology (for example, 70% or higher, such as 70%, 75%, 80%, 85%, 90%, 95%, or even 98%, 99%, or 100%) with the sequence of the present disclosure (preferably a sequence shown in any one of SEQ ID NO.: 6-10) is also encompassed within the scope of the present disclosure, as long as those skilled in the art can readily isolate such a sequence from other plants based on the information provided by the present application after reading the present application. A method and a tool for aligning sequences to determine an identity are also well known in the art, such as BLAST.

The polynucleotide of the present disclosure may be in a DNA or RNA form. The DNA form includes DNA, genomic DNA, or synthetic DNA. DNA can be single-stranded or double-stranded. DNA may be either a coding strand or a non-coding strand. A coding sequence encoding a mature polypeptide may be identical to a coding sequence of a sequence shown in any one of SEQ ID NO.: 6-10, or may be a degenerate variant thereof.

A polynucleotide encoding a mature polypeptide includes: a coding sequence that encodes only the mature polypeptide, a coding sequence for the mature polypeptide and various additional coding sequences; and a coding sequence for the mature polypeptide (and an optional additional coding sequence) and a non-coding sequence.

The term "polynucleotide encoding a polypeptide" may refer to a polynucleotide encoding the polypeptide, and may also be a polynucleotide including an additional coding and/or non-coding sequence. The present disclosure also relates to variants of the polynucleotides mentioned above, and these variants encode fragments, analogs, and derivatives of polynucleotides or polypeptides with the same amino acid sequences as those in the present disclosure. The variants of the polynucleotides can be either natural allelic variants or non-natural variants. These variants of the polynucleotides include substitution variants, deletion variants, and insertion variants. As known in the art, an allelic variant is an alternative form of a polynucleotide, which may involve the substitution, deletion, or insertion of one or more nucleotides without substantially changing a function of a polypeptide encoded accordingly.

The present disclosure also relates to polynucleotides that can hybridize with the above sequences and have an identity of at least 50%, preferably at least 70%, and more preferably at least 80% with the sequences, respectively. The present disclosure particularly relates to polynucleotides that can hybridize with the polynucleotides in the present disclosure under stringent conditions. In the present disclosure, the "stringent conditions" are as follows: (1) The hybridization and elution are conducted at a relatively-low ion intensity and a high temperature, such as 0.2 × SSC, 0.1% SDS, and 60°C. Alternatively, (2) A denaturing agent is added during hybridization, such as 50% (v/v) formamide, 0.1% calf serum/0.1% Ficoll, and 42°C. Alternatively, (3) The hybridization occurs only when an identity between two sequences is at least 90% or higher and preferably 95% or more.

A full-length nucleotide sequence or fragment of the FAD2-1A gene, the FAD2-1B gene, the Lox1 gene, the Lox2 gene, or the Lox3 gene of the present disclosure can generally be produced through PCR amplification, recombination, or artificial synthesis. For the PCR amplification, primers can be designed according to the relevant nucleotide sequence disclosed in the present disclosure, especially an open reading frame sequence, and a commercially-available DNA library or a cDNA library prepared by the conventional method known to those skilled in the art can be used as a template for amplification to produce the relevant sequence. When a sequence is long, two or more PCR amplifications often need to be conducted, and then amplified fragments are spliced together in a correct order. Once a relevant sequence is acquired, the relevant sequence can be produced in large quantities through recombination. Generally, the relevant sequence can be cloned into a vector and then transformed into a host cell, and then the related sequence can be isolated from the proliferated host cell by the conventional method.

The relevant sequences can also be synthesized artificially, especially when a fragment length is small. Typically, a plurality of small fragments are first synthesized and then ligated to produce a long fragment. Currently, a DNA sequence encoding the protein (or a fragment thereof or a derivative thereof) of the present disclosure can be produced completely through chemical synthesis. Then the DNA sequence can be introduced into various known DNA molecules (or vectors) and cells in the art. In addition, a mutation can be introduced into the protein sequence of the present disclosure through chemical synthesis.

### Polypeptides encoded by the FAD2 genes

As used herein, the terms "fatty acid desaturase", "polypeptide encoded by the FAD2 gene", and "protein encoded by the FAD2-1A or FAD2-1B gene" can be used interchangeably, and all refer to a polypeptide of a fatty acid desaturase derived from a plant (such as soybean) and a variant thereof. In a preferred embodiment, a typical amino acid sequence of the polypeptide of the present disclosure is shown in SEQ ID NO.: 1 or 2.

The present disclosure also includes a polypeptide or a protein that has a homology of 50% or more (preferably 60% or more, 70% or more, and 80% or more, more preferably 90% or more, even more preferably 95% or more, and most preferably 98% or more, such as 99% or 100%) with the sequence shown in SEQ ID NO.: 1 or 2 of the present disclosure and exhibits the same or similar function as or to the sequence.

The term "same or similar function" primarily refers to possessing a fatty acid desaturase activity.

The present disclosure also encompasses a fragment and an analog of the fatty acid desaturase protein with the fatty acid desaturase activity. As used herein, the terms "fragment" and "analog" refer to polypeptides that essentially maintain the same biological function or activity as the natural fatty acid desaturase protein of the present disclosure.

### Polypeptides encoded by the Lox genes

As used herein, the terms "lipoxygenase", "polypeptide encoded by the Lox gene", and "protein encoded by the Lox1 or Lox2 or Lox3 gene" can be used interchangeably, and all refer to a polypeptide of a lipoxygenase derived from a plant (such as soybean) and a variant thereof. In a preferred embodiment, a typical amino acid sequence of the polypeptide of the present disclosure is shown in any one of SEQ ID NO.: 3-5.

The present disclosure also includes a polypeptide or a protein that has a homology of 50% or more (preferably 60% or more, 70% or more, and 80% or more, more preferably 90% or more, even more preferably 95% or more, and most preferably 98% or more, such as 99% or 100%) with a sequence shown in any one of SEQ ID NO.: 3-5 of the present disclosure and exhibits the same or similar function as or to the sequence.

The term "same or similar function" primarily refers to possessing a lipoxygenase activity.

The present disclosure also encompasses a fragment and an analog of the lipoxygenase protein with the lipoxygenase activity. As used herein, the terms "fragment" and "analog" refer to polypeptides that essentially maintain the same biological function or activity as the natural lipoxygenase protein of the present disclosure.

The polypeptide of the present disclosure can be a recombinant polypeptide, a natural polypeptide, or a synthetic polypeptide. The polypeptide of the present disclosure may be a naturally purified product or a chemically synthesized product, or can be produced by a recombination technique in a prokaryotic or eukaryotic host (such as bacteria, yeast, higher plants, insects, and mammalian cells). Depending on a host adopted for a recombinant production scheme, the polypeptide of the present disclosure may be either glycosylated or non-glycosylated. The polypeptide of the present disclosure may also include or exclude an initiating methionine residue.

The polypeptide fragment, derivative, or analog of the present disclosure may be: (i) a polypeptide with one or more conservative or non-conservative amino acid residues (preferably conservative amino acid residues) substituted, where the substituted amino acid residues may or may not be encoded by a genetic code; or (ii) a polypeptide with a substituent in one or more amino acid residues; or (iii) a polypeptide produced by fusing a mature polypeptide to another compound (such as, a compound that extends a half-life of the polypeptide, such as polyethylene glycol); or (iv) a polypeptide produced by fusing an additional amino acid sequence to a polypeptide sequence (for example, a leader sequence, a secretory sequence, a sequence or a proprotein sequence to purify the polypeptide, or a fusion protein). The fragments, derivatives, and analogs defined herein all fall within the scope well known to those skilled in the art.

In the present disclosure, the polypeptide variant is a derivative sequence produced through the substitution or deletion of several (typically 1 to 60, preferably 1 to 30, more preferably 1 to 20, and most preferably 1 to 10) or the addition of at least one amino acid, such as the addition of one or more (typically 20 or less, preferably 10 or less, and more preferably 5 or less) amino acids at a C terminus and/or an N terminus, based on an amino acid sequence shown in any one of SEQ ID NO.: 1-5. For example, when a substitution is conducted using an amino acid with similar or close properties in the protein, a function of the protein typically does not change. The addition of one or more amino acids at a C terminus and/or a terminus usually does not change the function of the protein. These conservative variations are preferably generated through the substitutions in Table 1.

**Table 1**

| Initial residue | Representative substitution | Preferred substitution |
|---|---|---|
| Ala(A) | Val;Leu;Ile | Val |
| Arg(R) | Lys;Gln;Asn | Lys |
| Asn(N) | Gln;His;Lys;Arg | Gln |
| Asp(D) | Glu | Glu |
| Cys(C) | Ser | Ser |
| Gln(Q) | Asn | Asn |
| Glu(E) | Asp | Asp |
| Gly(G) | Pro;Ala | Ala |
| His(H) | Asn;Gln;Lys;Arg | Arg |
| Ile(I) | Leu;Val;Met;Ala;Phe | Leu |
| Leu(L) | Ile;Val;Met;Ala;Phe | Ile |
| Lys(K) | Arg;Gln;Asn | Arg |
| Met(M) | Leu;Phe;Ile | Leu |
| Phe(F) | Leu;Val;Ile;Ala;Tyr | Leu |
| Pro(P) | Ala | Ala |
| Ser(S) | Thr | Thr |
| Thr(T) | Ser | Ser |
| Trp(W) | Tyr;Phe | Tyr |
| Tyr(Y) | Trp;Phe;Thr;Ser | Phe |
| Val(V) | Ile;Leu;Met;Phe;Ala | Leu |

The present disclosure also encompasses analogs of the claimed proteins. Differences between these analogs and the natural sequence shown in any one of SEQ ID NO.: 1-5 may refer to a difference in an amino acid sequence, or a difference in a modification form that does not affect sequences, or both of the former two. The analogs of the proteins include natural or induced genetic variants. The induced variants can be produced through various techniques, such as random mutagenesis induced by the radiation or exposure to a mutagenic agent, site-directed mutagenesis, or other known techniques of molecular biology. The analogs also include analogs with residues different from natural L-amino acids (such as D-amino acids), and analogs with non-natural or synthetic amino acids (such as β- and γ-amino acids). It should be understood that the proteins of the present disclosure are not limited to the representative proteins listed above.

The modification form (which typically does not change the primary structure) includes chemical derivatization for proteins *in vivo* or *in vitro,* such as acetylation or carboxylation. The modification may also include glycosylation, such as glycosylation modification during the synthesis and processing of a protein. The modification can be accomplished by exposing a protein to a glycosylase (such as a mammalian glycosylase or deglycosylase). The modification form also includes a sequence with a phosphorylated amino acid residue (such as phosphotyrosine, phosphoserine, and phosphothreonine).

### Agricultural inhibitors

The active substance of the present disclosure (such as an inhibitor for the lipoxygenase and fatty acid desaturase genes or the proteins encoded thereby) can be prepared into an agricultural formulation by the conventional method, such as a solution, an emulsion, a suspension, a powder, a foam, a paste, a granule, an aerosol, a natural and synthetic material impregnated with the active substance, a microcapsule in a polymer, and a seed coating agent.

The formulation can be produced by a known method. For example, the active substance is mixed with an extender. The extender is a liquid or liquefied gas or solid diluent or carrier, and can optionally be a surfactant, namely, an emulsifier and/or a dispersant and/or a foam-forming agent. For example, when water is adopted as the extender, an organic solvent can also be adopted as an additive.

The use of a liquid solvent as a diluent or a carrier is generally applicable, including aromatic hydrocarbons, such as xylene, toluene, or alkyl naphthalene; chlorinated aromatic hydrocarbons or chlorinated aliphatic hydrocarbons, such as chlorobenzene, vinyl chloride, or dichloromethane; aliphatic hydrocarbons, such as cyclohexane or paraffin, like mineral oil fractions; alcohols, such as ethanol or ethylene glycol and ethers and esters thereof; ketones, such as acetone, methyl ethyl ketone, methyl isobutyl ketone, or cyclohexanone; or less common polar solvents, such as dimethylformamide and dimethyl sulfoxide (DMSO), and water.

A liquefied gas diluent or carrier refers to a liquid that becomes a gas at room temperature and atmospheric pressure, for example, an aerosol propellant such as a halogenated hydrocarbon, and butane, propane, nitrogen, and carbon dioxide.

A solid carrier can be a ground natural mineral, such as kaolin, clay, talc, quartz, activated white clay, montmorillonite, or diatomaceous earth, and a ground synthetic mineral, such as highly dispersed silica, alumina, and silicates. A solid carrier for granules is crushed and graded natural zircon, such as calcite, marble, pumice, sepiolite, and dolomite; particles synthesized from inorganic and organic coarse powders; and organic materials such as particles of sawdust, coconut shells, corn stalks, and tobacco stems.

Nonionic and anionic emulsifiers may be adopted as emulsifiers and/or foam-forming agents, for example, polyoxyethylene-fatty acid esters and polyoxyethylene-fatty alcohol ethers, such as alkylaryl polyglycol ethers, alkyl sulfonate esters, alkyl sulfate esters, aryl sulfonate esters, and hydrolysis products of albumin. A dispersant includes, for example, a lignin sulfite waste liquid and methyl cellulose.

A binder can be adopted in the formulation, such as carboxymethyl cellulose, and natural and synthetic polymers in a powder, granule, or emulsion form, including gum arabic, polyvinyl alcohol, and polyvinyl acetate.

A coloring agent can also be adopted, for example, an inorganic dye, such as iron oxide, cobalt oxide, and Prussian blue; an organic dye, such as an organic dye, such as an azo dye or a metal phthalocyanine dye; and a trace nutrient agent, such as salts of iron, manganese, boron, copper, cobalt, aluminum, and zinc.

In the present disclosure, the term "agricultural formulation" generally refers to an agricultural plant growth regulator, including: an inhibitor for the lipoxygenase and fatty acid desaturase genes or the proteins encoded thereby as an active ingredient for improving a trait of a plant (for example, reducing a beany flavor of the plant), and an agriculturally-acceptable carrier.

As used herein, the term "agriculturally-acceptable carrier" refers to an agriculturally-acceptable solvent, suspending agent, or excipient for delivering the active substance of the present disclosure to a plant. The carrier can be a liquid or a solid. The agriculturally-acceptable carrier suitable for the present disclosure is selected from the group consisting of water, a buffer, DMSO, a surfactant such as Tween-20, or a combination thereof. Any agriculturally-acceptable carrier known to those skilled in the art can be adopted in the present disclosure.

The agricultural inhibitor of the present disclosure may include an agricultural composition.

The agricultural formulation of the present disclosure may be used in combination with other substances for reducing a beany flavor of a plant. The other substances for reducing a beany flavor can be plant growth regulators known to those skilled in the art.

The agricultural formulation of the present disclosure can be in various dosage forms, as long as the active ingredient can be effectively delivered into a plant. From the perspective of easy preparation and application, the preferred agricultural formulation is a spray or solution formulation.

The agricultural formulation of the present disclosure generally includes the active ingredient of the present disclosure at a content 0.0001 wt% to 99 wt% and preferably 0.1 wt% to 90 wt% of a total weight of the agricultural formulation. A concentration of the active ingredient of the present disclosure in a commercial formulation or a dosage form can vary widely. The concentration of the active ingredient of the present disclosure in a commercial formulation or a dosage form can be 0.0000001% to 100% (g/v) and preferably 0.0001% to 50% (g/v).

The sequences involved in the present disclosure are as follows:

| Sequence No. (SEQ ID No.) | Sequence description |
|---|---|
| 1 | Amino acid sequence of GmFAD2-1A |
| 2 | Amino acid sequence of GmFAD2-1B |
| 3 | Amino acid sequence of GmLox 1 |
| 4 | Amino acid sequence of GmLox2 |
| 5 | Amino acid sequence of GmLox3 |
| 6 | Nucleotide sequence of GmFAD2-1A |
| 7 | Nucleotide sequence of GmFAD2-1B |
| 8 | Nucleotide sequence of GmLox1 |
| 9 | Nucleotide sequence of GmLox2 |
| 10 | Nucleotide sequence of GmLox3 |

### Major advantages of the present disclosure:

The present disclosure has discovered for the first time that the inhibition on the expression levels or activities of lipoxygenase and fatty acid desaturase genes or proteins encoded thereby can significantly reduce a content of a beany-flavor compound or reduce a beany flavor, and particularly can remarkably reduce the contents of hexanal, 2-hexenal, 1-hexanol, and 1-octen-3-ol, thereby enabling the preparation of beany flavor-free soybean.

### DESCRIPTION OF THE DRAWING

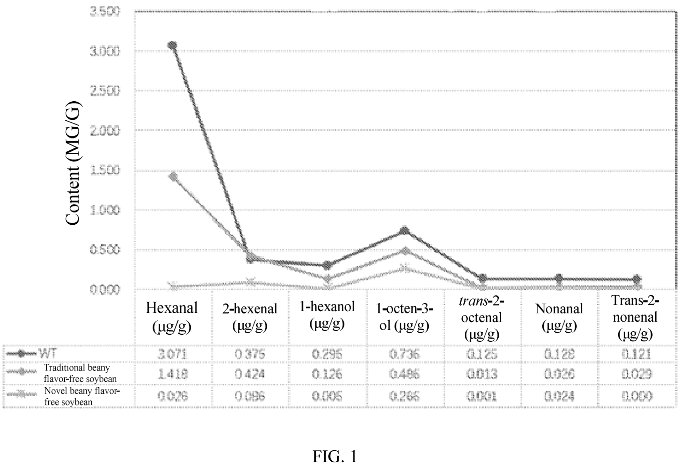

FIG. 1 shows the comparison of contents of beany-flavor compounds in soybean (the new-type beany flavor-free soybean in FIG. 1) of an experimental group (five mutations of fad2-1a/b and lox1/2/3), soybean (the traditional beany flavor-free soybean in FIG. 1) of a control group (three mutations of lox1/2/3), and WT soybean seeds.

### IMPLEMENTATIONS

The present disclosure will be further explained below in conjunction with an example. The following example is only a preferred example of the present disclosure, and is not intended to limit the present disclosure in other forms. Any technical personnel familiar with the profession may use the technical content disclosed above to derive equivalent examples through equivalent changes. Any simple modification or equivalent change made to the following example according to the technical essence of the present disclosure without departing from the content of the solutions of the present disclosure shall fall within the protection scope of the present disclosure.

### Example 1: Preparation of a gene-edited soybean line

### 1. Construction of a gene-editing vector

Genomic sequences and amino acid sequences of the GmFAD2-1A (Glyma10g42470), GmFAD2-1B (Glyma20g24530), GmLox1 (Glyma.13g347600), GmLox2 (Glyma.13g347500), and GmLox3 (Glyma.15g026300) genes were acquired from the National Center for Biotechnology Information (NCBI) (https://www.ncbi.nlm.nih.gov) website, and targets were designed specifically for coding regions of the five genes. In this example, soybean (Xudou 18) was subjected to gene editing by a CRISPR-based gene editing technology. A Cas protein adopted accordingly was a modified Cas12i protein (the Cas mutant protein S7R documented in the patent CN114672473B).

Based on the coding sequences of the GmFAD2-1A, GmFAD2-1B, GmLox1, GmLox2, and GmLox3 genes in soybean, gRNAs targeting Cas12i were designed. The designed gRNA sequences were as follows:

| gRNA | Guide sequence of gRNA (5' to 3') | PAM | Targeted gene |
|---|---|---|---|
| gRNA1 | cctcattgcatggccaatctat | ttc | GmFAD2-1A/B |
| gRNA2 | tagaaggttgcttcctgagaaag | ttc | GmLox1 |
| gRNA3 | ctcatcaacaccactgtcccttt | ttc | GmLox2 |
| gRNA4 | ttgcgcatcaacaccactgtccc | ttc | GmLox3 |

A direct repeat for the gRNAs was agagaaugugugcauagucacac (5' to 3'). The gRNA1 to gRNA4 each included the direct repeat and a respective guide sequence sequentially from 5' to 3'.

Annealing primers were designed based on the targets, and sequences of the annealing primers were as follows:

| | |
|---|---|
| GmFAD2-1A/B | acaccctcattgcatggccaatctat |
| | ggccatagattggccatgcaatgagg |
| GmLox1/2 | acactagaaggttgcttcctgagaaag |
| | ggccctttctcaggaagcaaccttcta |
| GmLox1/2 | acacctcatcaacaccactgtcccttt |
| | GGCCaaagggacagtggtgttgatgag |
| GmLox3 | acacttgcgcatcaacaccactgtccc |
| | ggccgggacagtggtgttgatgcgcaa |

After primer annealing, a gene editing backbone vector was ligated through Golden Gate assembly to produce the gene-editing vector. The gene-editing vector carried a Cas12i protein and the gRNAs.

A vector in an experimental group targeted the soybean genes GmFAD2-1A, GmFAD2-1B, GmLox1, GmLox2, and GmLox3 to achieve the simultaneous deletion of the five genes of GmFAD2-1A, GmFAD2-1B, GmLox1, GmLox2, and GmLox3. In addition, a three-mutation control group was set. A vector in the three-mutation control group targeted the soybean genes GmLox1, GmLox2, and GmLox3 to achieve the simultaneous deletion of the three genes of GmLox1, GmLox2, and GmLox3.

### 2. Acquisition of a recombinant strain

### 1) Transformation into Escherichia coli

The gene-editing vector obtained in the step 1 was transformed into *Escherichia coli,* and transformed *Escherichia coli* was subjected to bacterial-solution PCR. An amplification product with a correct PCR band size was selected for sequencing. *Escherichia coli* with a correct sequencing result was a recombinant *Escherichia coli* strain carrying the gene-editing vector.

### 2) Transformation into Agrobacterium

The recombinant *Escherichia coli* strain carrying the gene-editing vector obtained in the step 1) was cultured. Then the plasmid DNA was extracted, added to *Agrobacterium* competent cells, and placed in an ice bath for 5 min, in liquid nitrogen for 5 min, in a water bath at 37°C for 5 min, and on ice for 5 min.

A centrifuge tube was taken out, 700 µL of a medium (without an antibiotic) was added, and shaking culture was conducted at 28°C for 2 h to 4 h.

A resulting bacterial solution was collected and coated on a medium plate including a corresponding antibiotic, and invertedly cultured in an incubator. After about 2 d of the culturing, colonies could be observed. The colonies were subjected to PCR according to the method in the step 1). An amplification product was sequenced. *Agrobacterium* with a correct sequencing result was a recombinant *Agrobacterium* strain carrying the gene-editing vector.

### 3. Genetic transformation of a plant

### 1) Sterilization of soybean seeds:

The selected soybean seeds were placed in a petri dish in a dryer, 10 mL of concentrated hydrochloric acid was slowly added along a wall to a beaker with 150 mL of sodium hypochlorite in the dryer, and a lid of the dryer was quickly closed. The sterilization was conducted for 16 h with a chlorine gas generated from the sodium hypochlorite and concentrated hydrochloric acid.

### 2) Seed germination:

The sterilized seeds obtained in the step 1) were inserted into an MS medium with a hypocotyl facing downwards and an insertion depth being 1/3 to 1/2 of a seed width, and incubated overnight at 25°C in the dark or under light.

### 3) Preparation of an Agrobacterium suspension:

A bacterial solution stored in a -80°C freezer was taken, streaked (the recombinant *Agrobacterium* strain carrying the gene-editing vector obtained in the step 2) of 2) on an antibiotic-containing YEP plate, and cultured at 28°C for 2 d. Colonies were picked and inoculated into 5 mL of a YEP liquid medium in a 50 mL centrifuge tube, and cultured overnight at 28°C under shaking. 300 µL of a resulting bacterial solution was taken and added to 250 mL of a YEP liquid medium, and cultured overnight until OD₆₀₀ was about 0.6. Centrifugation was conducted at 4,000 rpm for 10 min. Dilution was conducted with an infection solution to OD₆₀₀ of about 0.6 for later use.

### 4) Cotyledonary node scratching, infection, and co-culture:

1 d after the seed germination in the step 2), a transverse incision was created on a hypocotyl at a position 3 mm to 5mm near a cotyledonary node, a longitudinal incision was created between two cotyledons without damaging the cotyledons, the hypocotyl was longitudinally split, an apical bud was removed, and 1 to 5 longitudinal incisions were created lightly on a cotyledonary node. Then seeds were quickly placed in the *Agrobacterium* infection solution prepared in advance in the step 3), infected for about 1 h to 4 h, then taken out and placed on a co-culture medium covered with a filter paper, and co-cultured at 22°C for 3 d to 5 d.

### 5) Recovery culture:

An explant obtained after the co-culture was inserted into a recovery medium in a petri dish, the petri dish was sealed with a 3 M surgical tape and placed under light, and the recovery culture was conducted at about 25°C for 5 d to 7 d.

### 6) Screening culture:

A recovered explant was inserted into the screening medium with a wound immersed, and screening was then conducted at about 25°C under light. Subculturing was conducted once every 10 d with 3 times to 5 times in total.

### 7) Bud elongation culture:

Cotyledons were removed from the selected explant, and a cluster bud-containing portion was transferred to the bud elongation medium and cultured. Subculturing was conducted once every 10 d. The bud elongation culture was conducted under the same light and temperature as the screening culture.

### 8) Rooting and seedling transplanting:

When seedlings in the bud elongation culture grew to 3 cm or more (or there were two trifoliate leaves), the seedlings were cut along a base, inserted into a sterilized nutrition soil, and then irrigated with a rooting nutrient solution to induce the root growth. Upon root emergence, resulting seedlings were transferred to a soil watered thoroughly in advance, and covered with a plastic film. One week later, the plastic film was cut open, seedlings were exercised. Two weeks later, if new leaves grew, the plastic film was completely removed to obtain E0 transformed seedlings.

### 4. Detection and phenotypic observation for transformed soybean plants

Edited seedlings were detected and selected from the E0 transformed seedlings through PCR and sequencing, and planted in a climate chamber.

### 5. Results

Conditions of the edited soybean plants were detected. According to results, in the experimental group (five mutations), sequences of the soybean genes GmFAD2-1A, GmFAD2-1B, GmLox1, GmLox2, and GmLox3 were partially deleted or inserted with amino acids, and functions of the genes completely lost. In the three-mutation control group, functions of the genes GmLox1, GmLox2, and GmLox3 completely lost, but the genes GmFAD2-1A and GmFAD2-1B had normal functions and underwent no editing events.

### Phenotypic identification for the edited soybean plants:

WT soybean seeds and soybean seeds of E1 edited plants (the experimental group and the three-mutation control group) were crushed and then tested for contents of beany-flavor compounds (hexanal, 2-hexenal, 1-hexanol, 1-octen-3-ol, *trans-2-octenal,* nonanal, and *trans*-2-nonenal). The detection of the contents of the beany-flavor compounds could be carried out by the conventional technique in the art.

Results were shown in FIG. 1. Compared to the WT soybean seeds (WT in FIG. 1), the contents of beany-flavor compounds in the soybean seeds of the three-mutation control group (the traditional beany flavor-free soybean in FIG. 1) significantly decreased (except for hexanal and 2-hexenal), and the contents of beany-flavor compounds in the soybean seeds (the new-type beany flavor-free soybean in FIG. 1) in the experimental group (five mutations) significantly decreased. Compared to the soybean seeds in the three-mutation control group, the contents of all beany-flavor compounds in the soybean seeds of the experimental group (five mutations) significantly decreased, especially hexanal, 2-hexenal, 1-hexanol, and 1-octen-3-ol. Specifically, a content of hexanal was reduced by 98% or more, a content of 1-hexanol was reduced by 96% or more, and contents of 2-hexenal and 1-octen-3-ol were reduced by about 80% and 50%, respectively. Although the contents of beany-flavor compounds in the soybean seeds of the three-mutation control group were relatively low, the soybean seeds of the experimental group (five mutations) included almost no beany-flavor compounds (the contents of various beany-flavor compounds detected were mostly lower than 0.09 µg/g, and some beany-flavor compounds even completely disappeared).

Although the specific implementations of the present disclosure have been described in detail, those skilled in the art will understand that various modifications and changes can be made to the details according to all teachings published, and such modifications and changes all are within the protection scope of the present disclosure. The full content of the present disclosure is defined by the appended claims and any equivalents thereof.

## Claims

1. A method for reducing a content of a beany-flavor compound in a plant or reducing a beany flavor of the plant, comprising a step of reducing or inhibiting expression levels and/or activities of lipoxygenase and fatty acid desaturase genes or proteins encoded thereby in the plant,
wherein the lipoxygenase gene is selected from any one or more of a Lox1 gene, a Lox2 gene, or a Lox3 gene; and
the fatty acid desaturase gene is selected from an FAD2-1A gene and/or an FAD2-1B gene.

2. The method according to claim 1, wherein the beany-flavor compound is selected from the group consisting of hexanal, 2-hexenal, 1-hexanol, 1-octen-3-ol, *trans*-2-octenal, nonanal, *trans*-2-nonenal, or a combination thereof.

3. The method according to any one of claims 1 to 2, wherein the reducing or inhibiting expression levels and/or activities of lipoxygenase and fatty acid desaturase genes or proteins encoded thereby in the plant is achieved by a technology selected from the group consisting of gene mutation, gene knockout, gene disruption, an RNA interference technology, a gene editing technology, introduction of an inhibitor for the genes or the proteins, or a combination thereof.

4. The method according to any one of claims 1 to 3, wherein the plant is soybean.

5. A composition for reducing a content of a beany-flavor compound in a plant or reducing a beany flavor of the plant, comprising:
(a) an inhibitor for lipoxygenase and fatty acid desaturase genes or proteins encoded thereby in the plant; and optionally,
(b) an agriculturally-acceptable carrier,
wherein the lipoxygenase gene is selected from any one or more of a Lox1 gene, a Lox2 gene, or a Lox3 gene;
the fatty acid desaturase gene is selected from an FAD2-1A gene and/or an FAD2-1B gene;
preferably, the inhibitor is selected from the group consisting of a gene editing reagent, an antisense nucleic acid, an antibody, a small-molecule compound, a Crispr reagent, a small-molecule ligand, or a combination thereof; and
preferably, the beany-flavor compound is selected from the group consisting of hexanal, 2-hexenal, 1-hexanol, 1-octen-3-ol, *trans*-2-octenal, nonanal, *trans*-2-nonenal, or a combination thereof.

6. A use of the composition according to claim 5 in reducing a content of a beany-flavor compound in a plant or reducing a beany flavor of the plant; or in preparation of a reagent or kit for reducing a content of a beany-flavor compound in a plant or reducing a beany flavor of the plant.

7. A method for preparing a plant cell, a plant seed, a plant tissue, a plant part, or a plant with a reduced content of a beany-flavor compound or a reduced beany flavor, comprising the following step:
reducing or inhibiting expression levels and/or activities of lipoxygenase and fatty acid desaturase genes or proteins encoded thereby in a plant cell, a plant seed, a plant tissue, a plant part, or a plant,
wherein the lipoxygenase gene is selected from any one or more of a Lox1 gene, a Lox2 gene, or a Lox3 gene;
the fatty acid desaturase gene is selected from an FAD2-1A gene and/or an FAD2-1B gene; and
preferably, the beany-flavor compound is selected from the group consisting of hexanal, 2-hexenal, 1-hexanol, 1-octen-3-ol, *trans-2-octenal,* nonanal, *trans*-2-nonenal, or a combination thereof.

8. A method for preparing a plant with a reduced content of a beany-flavor compound or a reduced beany flavor, comprising the following step:
regenerating a plant from the plant cell, the plant seed, the plant tissue, or the plant part prepared by the method according to claim 7, so as to obtain the plant with the reduced content of the beany-flavor compound or the reduced beany flavor,
wherein further, the method further comprises a step of harvesting a plant seed from the plant with the reduced content of the beany-flavor compound or the reduced beany flavor.

9. A method for improving a plant, comprising the following steps:
(a) providing a plant cell, a plant tissue, or a plant part; and introducing an inhibitor for lipoxygenase and fatty acid desaturase genes or proteins encoded thereby into the plant cell, the plant tissue, or the plant part, or reducing or inhibiting expression levels and/or activities of the lipoxygenase and fatty acid desaturase genes or the proteins encoded thereby in the plant cell, the plant tissue, or the plant part; and
(b) regenerating a plant from a plant cell, a plant tissue, or a plant part obtained in the step (a),
wherein the lipoxygenase gene is selected from any one or more of a Lox1 gene, a Lox2 gene, or a Lox3 gene;
the fatty acid desaturase gene is selected from an FAD2-1A gene and/or an FAD2-1B gene;
preferably, the improving a plant refers to reducing a content of a beany-flavor compound in the plant or reducing a beany flavor of the plant; and
preferably, the beany-flavor compound is selected from the group consisting of hexanal, 2-hexenal, 1-hexanol, 1-octen-3-ol, *trans-2-octenal,* nonanal, *trans*-2-nonenal, or a combination thereof.

10. The method according to any one of claims 7 to 9, wherein the plant is soybean.
